# EUROPEAN PATENT APPLICATION

(11) **EP 2 133 121 A1**
(43) Date of publication of application: **16.12.2009**
(21) Application number: 08010643.8
(22) Date of filing: 11.06.2008
(51) Int. Cl.: A61P 1/04, A61K 36/45, A61K 35/74, A61K 33/30, A23L 2/02

(54) **Nutritional additives containing cranberry extract for the digestive system and their uses**

(71) Applicant: Laboratoires SANTINOV, 42161 Andrezieux Boutheon cedex (FR)
(72) Inventor: The designation of the inventor has not yet been filed
(74) Representative: Burtin, Jean-François

(57) **Abstract**

This invention relates to the necessities of the life and namely to the nutrition.

It has as the subject matter a nutritional supplement intended to prevent or to counteract the recurring gastric discomfort which contain as the active ingredient a cranberry extract titrated in polymeric proanthocyanidines in a solid or liquid carrier suitable for oral administration.

Use of the said nutritional supplement in digestive surgery, to eliminate Helicobacter pylori in the digestive system, in order to contribute to sustain the normal secretion of gastric mucus and contribute to maintain the integrity of the mucosal protective barrier.

## Description

This invention relates to the necessities of the life and particularly to the field of health.

It has more particularly as subject matter nutritional additives susceptible to prevent or remedy to the disturbances linked to environmental and microbial problems.

More specifically it relates to novel nutritional additives intended to counteract the microbial infections associated with to the occurrence of gastric ulcer as well as the duodenal ulcer

It is known that the pathology bound to the detection and occurrence of an ulcer has been the subject of a significant increase linked as thought, to constraints or to the stress which generates a pepsic or hydrochloric acid hypersecretion

In accordance thereof for numerous years, ulcer has been treated administering neutralizing agents or adsorbent products such as insoluble salts of bismuth, aluminium salts, magnesium phosphate, titanium dioxide, intended to insure an efficient coverage of the gastric mucosa.

This therapy has only provided very limited and transient results. More recently the therapy of gastric ulcer has evoluted due to the fact that the administration of antihistaminic drugs has been the subject of various publications showing that the occurrence of ulcer might be involved with a hydrochloric acid hypersercretion under the control of histamine.

Several antihistaminics of the H₂ type such as cimetidine, ranitidine, famotidine have been used with some success in the treatment of ulcer.

A further progress has been achieved in the last ten years with the use of inhibitors of the proton pump which leads to a complete stopping of the hydrochloric acid secretion in the stomach. They may be cited for this purpose esomeprazole, omeprazole, lansoprazole or rabeprazole. This therapy is efficient but it is not without risk since one totally interrupts the hydrochloric acid secretion with gastric juice.

A novel step has been reached by administering antibiotics of the penicillin type such as amoxicillin, when considering that the treatment of ulcer essentially consists in the eradication of a microbial factor such as Helicobacter pylori which besides its pathogenic power, easily colonizes the digestive system and contributes to an increase of the gastric pH by virtue of its secretion of urease.

This statement has allowed to obtain a significant progress in the prevention or curing of ulcer while letting the deletion the main causal agent. It is considered at the present time that the essential factor responsible of the occurrence of gastric or duodenal ulcer resides in the fact that Helicobacter pylori proliferates and this important statement has arose the problem of the struggle against Helicobacter pylori which is a very resistant bacteria and against which very few antibiotics are even efficient.
Experiments have shown that penicillin are poorly active against Helicobacter and that extended treatments with such antibiotics leads only to the selection of species more and more resistant to antibiotics which lost there from about the wholeness of their efficiency.

It existed therefrom an important therapeutic problem for which no satisfactory solution existed as far as the causal agent of gastric or duodenal ulcer was clearly identified but against which not any really efficient mode of treatment was established.

The present invention on which this patent application lays resides in the fact that applicants have discovered that fluid or solid preparations based on cranberry (Vaccinium macrocarpon) constituted an efficient means for eliminating Helicobacter pylori.

The applicant has there turned itself .to a new utilisation of the anti adhesive properties of the extracts of cranberry.

Even if this proposal of anti-adhesion appeared to be efficient against Escherichia coli, it appears more and more the subject of publications on Helicobacter pylori, enterobacteria main ly responsible of the gastric or duodenal ulcers. However in contrast to the cases of cystitis the poly proanthocyanidines (PAC), the main ingredient of cranberry are not or are poorly utilized in the therapy of ulcer. It is known that their use is particularly interesting as nutritional means, as a substituant or addition to the usual therapy for the taking into account of the gastric ulcer, as it is today the case for cystitis and therefore it is an incitation to develop this indication.

The results of the achieved trials support the hypothesis along which this bacteria Helicobacter pylori, takes an active part for the diseases of the gastric mucosa as well as gastric ulcers in the upper part of the stomach and the intestines. Moreover it appears that the presence of these bacteria Helicobacter pylori could be an incentive for the risk of stomach cancer.

Taking account of the anti adhesive properties shown by Cranberries against Escherichia coli, searchers have studied the action of non-dialyzable materials of high molecular weight stemming from cranberries extracts in "in vitro" studies of adherency using three different strains of H. pylori having a specific effect on human gastric mucosa cells.

The results thereof have shown that this material inhibited the specific to sialic acid adherence to the human gastric mucosa in a dose-and strain-dependant manner. As the searchers estimated, a significant inhibition of the adherence appeared when strains of H. pylori have been incubated with the material stemming from cranberries but not when mucus alone was pre-incubated or when the material stemming from cranberry was added after the bacteria have adhered to the mucus.

More recently a more extensive study has tested the "in vitro" effects of non-dialyzable material on the inhibition of the adhering of 22 strains of H. pylori specific to human mucus and to a line of human gastric cells.

The data have shown that adherency specific to sialic acid of H. pylori is inhibited by non dialyzable material stemming from cranberry juice. At a concentration of 0,2 mg/ml non dialyzable material, 12 strains have been inhibited for more than 75%, 7 strains have been partially inhibited for 35 to 74% and only 3 strains were weakly inhibited.
Again the stripping of H. pylori from the mucus and from the epithelial cells has not been observed even at doses as high as 100 mg/ml, fixation of Helicobacter on the stomach or duodenum walls which are predilection places for the implantation of such a microbial germ.

It is also possible that the poly phenols contained in the compositions based on cranberry itself demonstrate antibacterial properties and contribute to the eradication of this microbial flora.

Moreover it has been observed that the addition of a bifidogenic agent such as inuline to the compositions based on cranberry has the effect of reinforcing the anti-Helicobacter action where the literature does not describe any property for this compound other than alimentary as a bifidogenic member.

Compositions according to this invention are therefore made as active ingredients acting for the suppression of the microbial infestation due to Helicobacter pylori with a synergic combination of extract of cranberry and a bifidogenic agent, in a solid or liquid carrier suitable for the administration as nutritional complement.

As cranberry extract it is understood any liquid or solid preparation obtained from the fruits of Vaccinium macrocarpon, optionally purified to eliminate sugars, bitter substances or any load without physiological effect.

It is also understood as cranberry preparation, concentrated juices of fruits of Vaccinium macrocarpon, as well as powdery products obtained by concentration or evaporation to dryness of such juices.

It is also understood with this term any powder resulting from the concentration or fractioning of the juices of fruits of Vaccinium macrocarpon to retain only the active phenolic fractions. It is in fact known that the compositions based on cranberries are constituted with poly phenols of proanthocyanidine type of the A group in the form of polymers such as for example trimers, tetramers, pentamers or hexamers.

The juices of fruits of Vaccinium macrocarpon contain significant amounts of proanthocyanidines up to 50 mg for 100 ml of juice. The proanthocyanidines as well as the tannic substances contribute to the efficiency of the compositions according to this invention as well as to provide an astringent taste belonging to substances related to tannin.

In the compositions according to the invention inuline or any bifidogenic substance is admixed in the powdery form. They are used as inuline the beet inuline a chicory inuline Jerusalem's artichoke inuline, dahlia roots inuline. Inuline maybe replaced by any other bifidogenic member which improves the characteristics of the intestinal flora and namely the fructo oligosaccharides. They act as saprophytic members of the bacterial flora.

The compositions of the invention contain also another constituent. It is the addition of zinc derivatives. With zinc derivatives it is understood any zinc salt, any zinc oxide or any complex with a zinc atom. The term zinc salt include any salt combined with a mineral acid such as zinc sulphate, zinc nitrate, zinc phosphate, or with an organic acid such as zinc acetate, zinc propionate, zinc benzoate, zinc salicylate, zinc gluconate, zinc lactobionate, zinc benzene sulfonate, zinc nicotinate, zinc pidolate, zinc acesulfamate, zinc glycinate, zinc mercapto-propionate, zinc citrate or zinc tartarate or any salt the anion of which is not harmful.

They are also understood under this term the various complexes of zinc with amino acids derivatives or amino compounds such as nitrilotriacetic acid, ethylene diamino tetrapropionic acid, ethylene diamino dihydroxytetra acetic acid, or ethylene diamino tetra acetic acid.

The amounts of zinc salts many vary depending on the nutritional supply into zinc salts. These amounts range from 0.1 mg to the maximal daily advised supply.

Instead of inuline it may also be used any plant which is called "carminative species" such as roman camomile, coriander, tarragon, fennel, common laurel (leaves) marjoram (leaves), papaya (leaves, juices), liquorice, thyme, the salvias, as well as anise, basil, blueberry, pepper mint, in the form of an oil or a part of a plant.

As already known the advised nutritional supplies in zinc are broadly dependant on the nutrition. Accordingly supplies of 2.5 mg or 3.6 mg in the adults (man or woman) may be sufficient in the case of a feeding with products of animal origin. In the case of more varied alimentation, supplies of the order of 6.5 mg/ day in the woman or 9.4 mg / day in the adult man are needed. However in the case where alimentation is rich in vegetables, an increase in the supply ranging up to 13 mg/ day in the woman and up to 18,5 mg / day in the adult man appears to be needed.

Therefore it is advised to use two different levels of nutritional supply into zinc depending on whether the feeding is relatively rich or at the contrary poor in products of animal origin.

In the frame of this invention, one utilizes zinc for the important role it plays in the normal secretion of mucus and for the preservation of the integrity of the mucosal protective barrier action according to that it has been disclosed by Furuta S. (Jpn J. Pharmacol. 1995 Apr. 67 (4) 271-278).

The nutritional complements according to the invention are preferably offered in the solid form, namely a powdery form such as in the form of sachets of powder. It contains from 500 to 2500 mg of titrated powders of Cranberry (titre of 1% proanthocyanidines determined according to DMAC method) i.e. a titer of 2% determined according the HPLC method - or from 100 to 200 mg of isolates of proanthocyanidines titrated at 10 to 20% of PAC using the DMAC method (i.e. 20 to 40 % using the HPLC method).

These complements also contain per unit dosage from 500 to 2500 mg bifidogenic agent such as inuline in the form of chicory roots powder (titrated at 90% inuline) or in the form of dalhia roots powder, Jerusalem's artichoke roots, or beet powder and further zinc in the form of a derivative for 4 to 6 mg zinc element.

The nutritional supplements according to this invention namely in the form of a powder may be sweetened, flavoured or aromatized to improve the flavour. They may also contain an inert diluent. The raspberry, strawberry or cherry flavours are perfectly convenient.

The nutritional supplements according to the invention find a very useful utilization in preventing or reducing the gastric recurrent discomfort, mainly as an anti-adherency agent against H. pylori, as a probiotic bifidogenic agent and as an essential metabolite of the normal secretion of mucus while maintaining the integrity of the barrier function of the mucosa of the digestive system.

### Dosology

1- In the presurgical operating phase, namely the surgery of obesity through stomach ring or by-pass.
   Ideally one must start the preparatory treatment immediately on making the appointment for the surgical operation, otherwise take every day during the 15 days preceding the surgical operation in accordance with the following scheme: the galenic formulation is designed in order to insure the integrity and activity of the acting ingredients as well as to optimize their effects. Therefore it has to be stressed on the mode of consumption of the products. Each dose bag must essentially be diluted in at least 250 ml drinkable water before to be absorbed (preferably weakly mineralized water).
   - From the 15th to 8th day before surgery: 2 bags per day, one hour before the two main meals.
   - From the 7th day to the day before the surgery: 3 bags per day one hour before the meals. It is advisable that the last allowed food intake for the patient before the surgical intervention would be a bag of the product according to the invention in order that the nutritional effect is maximal at the time of the intervention.
2- In the post operative surgical phase
   As soon as possible after the surgery, retake two bags per day one hour before the main meals for at least 15 days then change to one bag per day for at least 45 days. Thereafter regularly take treatments with one bag per day for 15 days.
3- In the phase of maintenance of the alimentary regimen. As a preventing mean in the predisposed individuals to gastric discomfort, make treatments for 15 days with 2 bags per day. Conclusively the nutritional composition according to the invention finds a use for its benefit full nutritional effect, or for its taking part to the eradication of Helicobacter pylori in the digestive system in the people which suffer from recurring gastric discomfort or which are subjected to a surgical intervention of gastric restriction (gastric ring) or of stomach malabsorption (by-pass and bilio-pancreatic derivation)

### Example:

### Sachets of powder according to the invention:

They are mixed until perfect homogeneity

| | |
|---|---|
| Cranberry powder titrating 1% proanthocyanidines | 500 g |
| Chicory inuline | 500 g |
| Zinc gluconate | 6 g |
| Lactose | 1000 g |
| Raspberry aroma | 0.2 g |
| For 1000 sachets of powder | |

Digestive system is an expression intended to define the whole parts of the digestion from the mouth, the throat, the stomach and the intestines up to the rectum.

## Claims

1. A nutritional supplement intended to prevent or to struggle the recurring gastric discomfort, wherein they contain as the active ingredient a cranberry extract titrated in polymeric proanthocyanidines in a solid or liquid carrient suitable for oral administration.

2. A nutritional supplement according to claim 1 wherein it further contains a bifidogenic agent and a zinc derivative.

3. A nutritional supplement according to claim 1 wherein the cranberry extracts is a juice or a concentrate of fruits of Vaccinium macrocarpon.

4. A nutritional supplement according to claim 1 and claim 2 wherein the cranberry extract contains from 500 to 2500 mg of polymeric proanthocyanidines per liter as determined by DMAC method or 100 to 200 mg per liter of isolate of polymeric proanthocyanidines as determined by DMAC method.

5. A nutritional supplement according to claim 1 and claim 2, wherein the bifidogenic agent is a fructo oligo saccharide.

6. A nutritional supplement according to the preceding claim wherein the fructo oligosaccharide is chicory inuline, dalhia roots inuline, Jerusalem's artichoke inuline or beet inuline.

7. A nutritional supplement according to claim 1 and claim 2 wherein the zinc derivative is an oxyde, a salt with a mineral or organic acid or a complex of zing.

8. A nutritional supplement according to any of the preceding claims wherein the organic acid addition salt is zinc gluconate, zinc glucuronate, or zinc lactobionate

9. A nutritional supplement according to claims 1 and claim 2, wherein the content of cranberry extract per unit dosage ranges from 500 to 2500 mg.

10. A nutritional supplement according to any of the preceding claims wherein the content on bifidogenic element per unit dosage ranges from 500 mg to 2500 mg in the form of inuline.

11. A nutritional supplement according to any of the preceding claims wherein the unit dosage of zinc element ranges from 4 to 6 mg.

12. A nutritional supplement according to any of the preceding claims in the form of a sachet of powder optionally aromatized, flavoured or diluted with an inert excipient.

13. Use of the nutritional supplement according to any of the preceding claims in digestive surgery on account of 1 to 3 sachets of powdery supplement per day.

14. Use of the nutritional supplement according to any of claims 1 to 12 as an additive for eliminates Helicobacter pylori in the digestive system.

15. Use of the nutrition supplement according to any of claims 1 to 12 as an important factor of the normal secretion of gastric mucus, and for maintaining the integrity of the mucosal protective barrier.

## Amended claims

### Amended claims in accordance with Rule 137(2) EPC.

**1.** A nutritional complement intended to prevent or counteract the recurring gastric disturbances wherein a mixture of cranberry extract titrated into anthocyanidin polymers of type A, a bifidogenic agent of the fructo oligosaccharide group and a zinc derivative is included.

**2.** A nutritional complement according to claim 1 wherein the content into proanthocyanidins is determined through the DMAC method.

**3.** A nutritional complement according to claim 1 wherein the fructo oligosaccharide is selected from the group consisting of chicorea inulin, inulin from dahlia roots, inulin from Jerusalem' s artichoke, and inulin from beets.

**4.** A nutritional supplement according to any of the preceding claims wherein the zinc derivative is an oxide, a mineral acid salt, an organic acid salt or a zinc complex.

**5.** A nutritional complement according to any of claims 1 to 4 wherein the zinc derivative is selected from the group consisting of zinc gluconate, zinc glucuronate and zinc lactobionate.

**6.** A nutritional complement according to any of claims 1 to 5 wherein the cranberry extract contains from 500 to 2500mg polymeric proanthocyanidins of type A per liter.

**7.** A nutritional complement according to any of the claims 1 to 6 wherein the complement is provided in the form of powder optionally flavoured or perfumed and diluted with an inert excipient.

**8.** Use of the nutritional supplement according to claim 1 in the surgery of the digestive tract at a rate of 1 to 3 sachets of powdery supplement per day.

**9.** Use of the nutritional supplement according to claim 1, in the elimination of Helicobacter pylori in the digestive tract at the rate of 2 sachets of powdery supplement per day.

**10.** Use of the nutritional supplement according to claim 1 in the preoperative or post operative surgical phase of the treatment of obesity at the rate of 2 to 3 sachets of the powdery composition of claim 1.
